# EUROPEAN PATENT SPECIFICATION

(11) **EP 2 102 183 B1**
(45) Date of publication and mention of the grant of the patent: **27.06.2012**
(21) Application number: 07857556.0
(22) Date of filing: 13.12.2007
(51) Int. Cl.: C07D 319/12

(54) **PRODUCTION OF CYCLIC DIESTERS OF ALPHA-HYDROXYACIDS**
HERSTELLUNG VON CYCLISCHEN DIESTERN VON ALPHAHYDROXYSÄUREN
PRODUCTION DE DIESTERS CYCLIQUES D'ALPHA-HYDROXYACIDES

(30) Priority: 13.12.2006 US 874475 P
(43) Date of publication of application: 23.09.2009
(73) Proprietor: Wajc, Samuel J., 34980 Haifa (IL)
(72) Inventor: Wajc, Samuel J., 34980 Haifa (IL)
(74) Representative: Jacques, Philippe
(86) International application number: PCT/EP2007/063909
(87) International publication number: WO 2008/071776

(56) References cited:
- WO-A-2006/124899
- US-A- 5 766 439
- J.PELOUZE: "Ueber die Milchsäure" JUSTUS LIEBIGS ANNALEN DER CHEMIE, vol. 53, 1845, pages 112-124, XP002483584

## Description

### BACKGROUND OF THE INVENTION

This Patent Application continues the Provisional Patent Application US60/874,475 with the same title.

A key intermediate in the production of polylactic acid (PLA) is the cyclic diester Lactide (LD).
After purification of the LD to rather stringent requirements, polymerization up to molecular weight of 400 000 by ring-opening in the presence of a homogeneous catalyst is relatively easy (US Pat. No. 5,319,107).

The production of LD itself by the classical process is more intricate:
a.- fermentation of a well-chosen carbohydrate in the presence of Ca(OH)₂ (or CaCO₃) leads to the production of a suspension of bacteria in a solution of Calcium Lactate (CaLac).
b.- the bacteria are separated by centrifugation or filtration and discarded (US Pat. No. 5,766,439)
c.- the filtrate reacts with Sulfuric Acid, which causes the precipitation of Gypsum (Calcium Sulfate Dihydrate) and the liberation of Lactic Acid (LA) as a solution of some 10% by weight.(US Pat. Appl. No. 20050281913)
d.- that solution is concentrated by distillation to 85-88% LA by weight.
e.- the concentrated LA solution, in the presence of a homogeneous catalyst, undergoes a prepolymerization in a vacuum distillation column, where more water is separated.
f.- as the molecular weight of the prepolymer is only about 1000, its mechanical properties are not suitable for industrial use.
g.- the prepolymer is then depolymerized by back-biting in the presence of a catalyst under vacuum and the LD leaving the reactor in the vapor phase is condensed as a liquid or directly fed to a distillation column to produce liquid crude LD (mixed with LA, some of its light oligomers, water, unwanted enantiomers of LD, etc...) (US Pat. No. 5,357,035).
h.- the crude LD is further purified by liquid-liquid extraction with water followed by crystallization from aqueous solution (US Pat. Appl. No. 20060014975).
i.- centrifugation gives a cake of purified LD, but since the impurity level is still too large, a last operation is required:
j.- melt crystallization with sweating to remove the impurities by gravity flow.

WO2006/124899 A2 discloses a method for producing lactide.

All these operations are well known, so that it is possible to produce for instance the enantiomer L-LD with a purity of up to 99.9%. But the yield of each of the operations of this long chain is rather modest, so that large recycle flows are required, so that the various equipment items tend to be large with large energy requirements.

In the classical process, a large amount of waste product (Calcium Sulfate Dihydrate, or gypsum) is produced.

Other alpha-hydroxyacids, such as glycolic acid, may similarly be dimerized to the corresponding cyclic diester and thence to the polyacid (e.g. glycolide leading to polyglycolic acid) by the same process and with the same disadvantages.

If it were possible to go from the Calcium Lactate directly to the LD, the production cost of PLA would decrease. This is the object of the present invention.

### BRIEF SUMMARY OF THE INVENTION

Since the whole classical process of LD production may be caracterized as a concatenation of ever more difficult dehydration steps, and since dehydration at temperatures larger than 200°C may lead to thermal degradation or to racemization of the LD, we have invented a process where the reactants introduced to the LD production reactor are themselves water absorbents, so that little or no water has to be evacuated, and so that by stoechiometry the only possible volatile reaction product is LD.

The invented process is the process which is claimed in appended claim 1, namely a process for the synthesis of cyclic diesters of alpha-hydroxyacids wherein:
(a) anhydrous alkalino-earth salts of the corresponding alpha-hydroxyacids are reacted, at room temperature, with a concentrated acid or anhydride selected from the group consisting of Sulfuric Anhydride, Sulfurous Anhydride, Oleum, Sulfuric Acid, Phosphoric Anhydride, Phosphoric Acid to form a pasty or solid mixture comprising an alkalino-earth salt of the acid,
(b) the reaction medium may be further partially dehydrated at a temperature that is low enough to prevent racemization or other damage to the organic product of interest, and
(c) the reaction medium may then be submitted either to solid-liquid extraction or to distillation-desublimation to recuperate the cyclic diester.

### DETAILED DESCRIPTION OF THE INVENTION

From the classical chain of operations, we retain only the first two ones:
A.- Fermentation of a well-chosen carbohydrate in the presence of Ca(OH)₂ (or CaCO₃) leads to the production of a suspension of bacteria in a solution of Calcium Lactate (CaLac).
B.- The bacteria are separated by centrifugation or filtration and discarded

The next three steps are similar to those disclosed in US Pat. No. 5,766,439:
C.- The filtrate (or centrate), a solution of Calcium Lactate, is concentrated by evaporation of water
D.- Cooling crystallization brings about separation of a hydrate of Calcium Lactate (the pentahydrate if the crystallization temperature is low enough)
E.- Separation of the crystals by centrifugation; further treatment of the mother-liquor for separation of more CaLac Pentahydrate (CaLac PH) and separation of a bleed solution.

The following steps embody the gist of the present invention:
F.- Dehydration of the Pentahydrate at atmospheric pressure and at a temperature smaller than 150°C to obtain anhydrous CaLac (Yukoho Sakata et al. 2005).
G.- Production of a "cement" by reaction at room temperature of the Calcium Lactate Anhydrate with a concentrated acid or anhydride chosen among Phosphoric Anhydride, Phosphoric Acid, Sulfuric Anhydride, Sulfurous Anhydride, Oleum, Sulfuric Acid. This reactant is chosen in such a way that after reaction with the anhydrous Calcium salt of the relevant alpha-hydroxyacid a mixture of solids is produced with as little as possible crystallization water. For instance, reaction of 1 mole of anhydrous CaLac with 1 mole SO₃ would lead (by stoechiometry) to a mixture of 1 mole LD,
   1 mole Calcium Sulfate and 1 mole water. This suggests that the anorganic salts would be a mixture of Calcium Sulfate Dihydrate (1/3) and Calcium Sulfate Hemihydrate (2/3).
H.- Heating the solid powder or granules to a temperature sufficient for reaction and evaporation of excess water at or slightly below atmospheric pressure. For instance, Gypsum (the Dihydrate) would loose 1.5 mole water per mole of gypsum around 125°C, too low a temperature for evaporation at atmospheric pressure of any LD or LA that might have been produced. In these conditions, i.e. in the absence of free water, no LA will remain. In practice, one slowly increases the temperature, taking care that no LA but only water appears in the condensate.
   I.- Sublimation (or rather distillation, since the melting point of L-LD is 98°C) of the LD from the mixture (for instance Calcium Sulfate Hemihydrate interspersed with LD) under vacuum or in the presence of inert gas. This is done only after setting of the "cement".
J.- Desublimation of LD as a cylindrical solid layer on the vertical tubes of a heat exchanger.
K.- Reheating this layer in order to induce "sweating" so that an impure viscous solution will be produced, but as opposed to what happens in melt crystallizers, the impurities will be evacuated by selective sublimation and not by gravity.
L.- The last step is similar to that in melt crystallization, i.e. evacuation of the purified crystals by complete melting of the layer.

It will be apparent to those skilled in the art that elimination of most of the water at a mild temperature, before production of LA and its dimerization is the key to this process, insofar as it allows the production of fairly concentrated LD dispersed in porous agglomerates of rather inert anorganic material. Lactide may well be molten, but capillarity keeps it inside the individual anorganic granules (or agglomerates of anorganic powder) if enough inert anhydrous powder keeps these granules (or agglomerates) apart. At this stage, it is possible to distill a vapour that is mainly LD, so that desublimation gives a rather pure product that may be further purified in the desublimator itself by sweating and sublimation (without having to move the product to another apparatus).

Notice that, apart from water that will be trapped by condensation after the desublimator, the waste product is now the hemihydrate of Calcium Sulfate, a waste product that may be used in the building industry.

Refering to the drawing, we see in the upper left corner the first apparatus of the plant, namely the fermentor F, fed with sugars, bacteria and nutrients in a manner known in the art. The bacteria present in the fermentation broth are separated in a decanter-centrifuge DC, the resulting mud being partly discarded and partly recycled to the fermentor.
The centrate is a fairly dilute solution (around 10%) of Calcium Lactate that is fed to an evaporator E, followed by a cooling crystallizer CC (alternatively, an evaporator-crystallizer may be used). Condensation water is discarded and the suspension of Calcium Lactate Pentahydrate is sent to a filter-centrifuge FC. The filtrate is recycled to the evaporator E, having been purified en route. The wet cake of the filter-centrifuge FC is dehydrated in the dryer DH1 and the resulting powder of CalciumLactate Anhydrate is fed to the reactor R. Gaseous Sulfuric Anhydride is fed to the same reactor, so that a mixture of LD, CaSO₄.2H₂O and CaSO₄.0.5H₂O is produced. This mixture is fed to a second dryer DH2, where the Calcium Sulfate Dihydrate is transformed in Hemihydrate (in order to limit the temperature to less than 98°C, this operation may be performed at atmospheric pressure in the presence of an inert gas or under a small vacuum). The resulting mixture is fed to a distillation apparatus DIST, operating at less than 200 °C, either under vacuum or in the presence of inert gas.
The distilled or entrained vapor will deposit the LD in the desublimator DES.

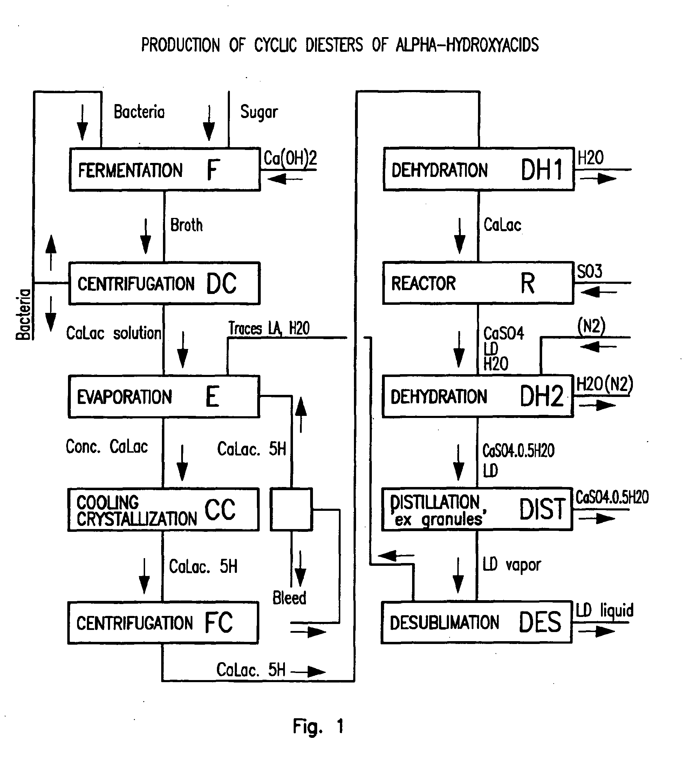

It will be apparent to those skilled in the art that Magnesium Lactate might have some advantage over Ca Lac. Its production by fermentation (US Pat. No. 3,429,777), the crystallization of its Trihydrate, the dehydration of the latter and the reaction with SO₃ are as easy as those of CaLac, and after reaction, one would produce Magnesium Sulfate Monohydrate (Kieserite), whose dehydration at 160°C is reputedly kinetically hindered, so that there would be no need for a step of partial dehydration (DH2 in fig.1) (Chipera, 2007).

It will also be apparent to those skilled in the art that the use of Phosphoric Anhydride
(a solid at room temperature) could lead, by reaction with anhydrous CaLac, to a mixture of Monocalcium Phosphate and Dicalcium Phosphate that might be suitable as fertilizer.

It will also be apparent to those skilled in the art that less volatile cyclic diesters could still be produced by a variant of the process described here above. Indeed, if separation by distillation and desublimation of the cyclic diester from the solid residue is not economically practical, it may still be possible to extract it (solid-liquid extraction) by a suitable solvent, such as toluene, at a temperature close to the diester melting point. This would be followed by crystallization from solution, separation by centrifugation, and drying of the cake.

## Claims

1. A process for the synthesis of cyclic diesters of alpha-hydroxyacids wherein:
(a) anhydrous alkalino-earth salts of the corresponding alpha-hydroxyacids are reacted, at room temperature, with a concentrated acid or anhydride selected from the group consisting of Sulfuric Anhydride, Sulfurous Anhydride, Oleum, Sulfuric Acid, Phosphoric Anhydride, Phosphoric Acid to form a pasty or solid mixture comprising an alkalino-earth salt of the acid,
(b) the reaction medium may be further partially dehydrated at a temperature that is low enough to prevent racemization or other damage to the organic product of interest, and
(c) the reaction medium may then be submitted either to solid-liquid extraction or to distillation-desublimation to recuperate the cyclic diester.

2. The process according to Claim 1, wherein the alpha-hydroxyacid is lactic acid.

3. The process according to Claim 2, comprising the following steps:
A.- Fermentation of a carbohydrate in the presence of Ca(OH)₂ or CaCO₃, leading to the production of a suspension of bacteria in a solution of Calcium Lactate (CaLac).
B.- Separation of the bacteria by centrifugation or filtration.
C.- Concentration of the filtrate (or centrate) by evaporation of water.
D.- Cooling crystallization to bring about separation of a hydrate of Calcium Lactate (the pentahydrate if the crystallization temperature is low enough) from a mother liquor.
E.- Separation of the crystals by centrifugation; further treatment of the mother-liquor for separation of more CaLac Pentahydrate (CaLac PH) and separation of a bleed solution.
F.- Dehydration of the Pentahydrate at atmospheric pressure and at a temperature smaller than 150°C to obtain anhydrous CaLac.
G.- Production of a "cement" by reaction at room temperature of the Calcium Lactate Anhydrate with a concentrated acid or anhydride chosen among Phosphoric Anhydride, Phosphoric Acid, Sulfuric Anhydride, Sulfurous Anhydride, Oleum, Sulfuric Acid.
H.- Heating the cement to a temperature sufficient for reaction and evaporation of excess water at or slightly below atmospheric pressure.
I.- Sublimation of the cyclic diester Lactide LD from the mixture under vacuum or in the presence of inert gas.
J.- Desublimation of LD as a cylindrical solid layer on the vertical tubes of a heat exchanger.
K.- Reheating this layer in order to induce "sweating" so that an impure viscous solution will be produced and that the impurities will be evacuated by selective sublimation.
L.- Evacuation of the purified crystals by complete melting of the layer.

4. The process according to Claim 2, wherein the alkalino-earth ion is Mg or Ca.

5. The process according to Claim 2, wherein the alkalino-earth ion is Mg and the concentrated acid or anhydride is SO3.

6. The process according to Claim 3, wherein the concentrated anhydride used in step G is Phosphoric Anhydride.

7. The process according to Claim 1 wherein the cyclic diester is separated from the solid mixture by solid-liquid extraction, followed by crystallization from solution, separation by centrifugation, and drying.

## Patentansprüche

1. Verfahren für die Synthese von cyclischen Diestern von alpha-Hydroxysäuren, wobei:
(a) wasserfreie Erdalkalisalze der entsprechenden alpha-Hydroxysäuren bei Raumtemperatur mit einer/einem konzentrierten Säure oder Anhydrid, ausgewählt aus der Gruppe bestehend aus Schwefelsäureanhydrid, Schwefligsäureanhydrid, Oleum, Schwefelsäure, Phosphorsäureanhydrid, Phosphorsäure, umgesetzt werden, um ein pastöses oder festes Gemisch zu bilden, umfassend ein Erdalkalisalz der Säure,
(b) das Reaktionsmedium ferner bei einer Temperatur, die ausreichend tief ist, um Racemisierung oder andere Beschädigung des organischen Produkts von Interesse zu verhindern, teilweise dehydriert werden kann, und
(c) das Reaktionsmedium anschließend entweder einer fest-flüssig-Extraktion oder einer Destillation-Desublimation unterzogen werden kann, um den cyclischen Diester zu gewinnen.

2. Verfahren gemäß Anspruch 1, wobei die alpha-Hydroxysäure Milchsäure ist.

3. Verfahren gemäß Anspruch 2, umfassend folgende Schritte:
A.- Fermentieren eines Kohlenhydrats in Gegenwart von Ca (OH)₂ oder CaCO₃, das zur Herstellung einer Suspension von Bakterien in einer Lösung von Calciumlactat (CaLac) führt,
B.- Abtrennen der Bakterien durch Zentrifugation oder Filtration,
C.- Konzentrieren des Filtrats (oder Zentrats) durch Abdampfen von Wasser,
D.- Kristallisieren durch Abkühlen, um Abtrennung eines Hydrats von Calciumlactat (des Pentahydrats, wenn die Kristallisationstemperatur tief genug ist) aus der Mutterlauge zu bewirken,
E.- Abtrennen der Kristalle durch Zentrifugation; Weiterbehandeln der Mutterlauge zum Abtrennen von weiterem CaLac-Pentahydrat (CaLac PH) und Abtrennen einer ausgezogenen Lösung,
F.- Dehydratisieren des Pentahydrats bei atmosphärischem Druck und einer Temperatur von unter 150°C, um wasserfreies CaLac zu erhalten,
G.- Herstellen eines "Zements" durch Umsetzen des Calciumlactat-Anhydrats mit einer/einem konzentrierten Säure oder Anhydrid, ausgewählt aus Phosphorsäureanhydrid, Phosphorsäure, Schwefelsäureanhydrid, Schwefligsäureanhydrid, Oleum, Schwefelsäure, bei Raumtemperatur,
H.- Erwärmen des Zements auf eine Temperatur, die für die Umsetzung und das Abdampfen von überschüssigem Wasser bei oder etwas unter atmosphärischem Druck ausreicht,
I.- Sublimieren des cyclischen Diesterlactids LD aus dem Gemisch unter Vakuum oder in Gegenwart eines inerten Gases,
J.- Desublimieren von LD als zylindrische feste Schicht auf den senkrechten Rohren eines Wärmeaustauschers,
K.- Wiedererwärmen dieser Schicht, um "Schwitzen" auszulösen, so dass eine verunreinigte viskose Lösung entsteht und die Verunreinigungen durch selektive Sublimation abgehen,
L.- Abfördern der gereinigten Kristalle durch vollständiges Schmelzen der Schicht.

4. Verfahren gemäß Anspruch 2, wobei das Erdalkali-Ion Mg oder Ca ist.

5. Verfahren gemäß Anspruch 2, wobei das Erdalkali-Ion Mg ist und die/das konzentrierte Säure oder Anhydrid SO₃ ist.

6. Verfahren gemäß Anspruch 3, wobei das bei Schritt G verwendete konzentrierte Anhydrid Phosphorsäureanhydrid ist.

7. Verfahren gemäß Anspruch 1, wobei der cyclische Diester durch fest-flüssig-Extraktion, gefolgt von Kristallisation aus Lösung, Abtrennen durch Zentrifugation und Trocknen von dem festen Gemisch abgetrennt wird.

## Revendications

1. Procédé de synthèse de diesters cycliques d'alpha-hydroxyacides dans lequel :
(a) des sels alcalino-terreux anhydres des alpha-hydroxyacides correspondants sont mis à réagir, à température ambiante, avec un acide ou anhydride concentré choisi dans le groupe constitué par l'anhydride sulfurique, l'anhydride sulfureux, l'oléum, l'acide sulfurique, l'anhydride phosphorique, l'acide phosphorique pour former un mélange pâteux ou solide comprenant un sel alcalino-terreux de l'acide,
(b) le milieu réactionnel peut être en outre partiellement déshydraté à une température qui est suffisamment basse pour empêcher la racémisation ou autre dégât au produit organique que l'on recherche, et
(c) le milieu réactionnel peut ensuite être soumis soit à une extraction solide-liquide, soit à une distillation-désublimation, pour récupérer le diester cyclique.

2. Procédé selon la revendication 1, dans lequel l'alpha-hydroxyacide est l'acide lactique.

3. Procédé selon la revendication 2, comprenant les étapes suivantes :
A. Fermentation d'un carbohydrate en présence de Ca(OH)₂ ou de CaCO₃, pour conduire à la production d'une suspension de bactéries dans une solution de lactate de calcium (CaLac).
B. Séparation des bactéries par centrifugation ou filtration.
C. Concentration du filtrat (ou surnageant) par évaporation de l'eau.
D. Cristallisation par refroidissement pour entraîner la séparation d'un hydrate de lactate de calcium (le pentahydrate si la température de cristallisation est suffisamment basse) à partir d'une liqueur mère.
E. Séparation des cristaux par centrifugation ; traitement plus poussé de la liqueur mère pour séparer davantage de pentahydrate de CaLac (CaLac PH) et séparation d'une solution de purge.
F. Déshydratation du pentahydrate à la pression atmosphérique et à une température inférieure à 150°C pour obtenir du CaLac anhydre.
G. Production d'un "ciment" par réaction à température ambiante du lactate de calcium anhydre avec un acide ou anhydride concentré choisi parmi l'anhydride phosphorique, l'acide phosphorique, l'anhydride sulfurique, l'anhydride sulfureux, l'oléum, l'acide sulfurique.
H. Chauffage du ciment à une température suffisante pour la réaction et l'évaporation de l'excès d'eau à la pression atmosphérique ou légèrement au-dessous.
I. Sublimation du lactide diester cyclique LD d'avec le mélange sous vide ou en présence de gaz inerte.
J. Désublimation du LD sous forme de couche solide cylindrique sur les tubes verticaux d'un échangeur de chaleur.
K. Réchauffage de cette couche afin de déclencher une "exsudation" de façon à produire une solution visqueuse impure et à faire en sorte que les impuretés soient évacuées par sublimation sélective.
L. Evacuation des cristaux purifiés par fusion complète de la couche.

4. Procédé selon la revendication 2, dans lequel l'ion alcalino-terreux est Mg ou Ca.

5. Procédé selon la revendication 2, dans lequel l'ion alcalino-terreux est Mg et l'acide ou anhydride concentré est SO₃.

6. Procédé selon la revendication 3, dans lequel l'anhydride concentré utilisé dans l'étape G est l'anhydride phosphorique.

7. Procédé selon la revendication 1 dans lequel le diester cyclique est séparé du mélange de solides par extraction solide-liquide, suivie d'une cristallisation dans la solution, d'une séparation par centrifugation et d'un séchage.
